# EUROPÄISCHE PATENTSCHRIFT

(11) **EP 3 177 337 B1**
(45) Veröffentlichungstag und Bekanntmachung des Hinweises auf die Patenterteilung: **10.07.2019**
(21) Anmeldenummer: 15739592.2
(22) Anmeldetag: 21.07.2015
(51) Int. Cl.: A61M 1/16, A61M 1/36

(54) **BLUTBEHANDLUNGSVORRICHTUNG MIT EINER FUNKTIONSEINHEIT ZUR DURCHFÜHRUNG DER BLUTBEHANDLUNG UND ZUR ÜBERPRÜFUNG DER FUNKTIONSFÄHIGKEIT UND/ODER DES BETRIEBSZUSTANDES DER FUNKTIONSEINHEIT**
BLOOD TREATMENT DEVICE COMPRISING A FUNCTIONAL UNIT FOR CARRYING OUT THE BLOOD TREATMENT AND FOR MONITORING THE FUNCTIONALITY AND/OR THE OPERATING STATE OF THE FUNCTIONAL UNIT
DISPOSITIF DE TRAITEMENT DU SANG COMPRENANT UNE UNITÉ FONCTIONNELLE SERVANT À EFFECTUER LE TRAITEMENT DU SANG ET SERVANT À SURVEILLER LA CAPACITÉ FONCTIONNELLE ET/OU L'ÉTAT DE FONCTIONNEMENT DE L'UNITÉ FONCTIONNELLE

(30) Priorität: 07.08.2014 DE 102014011695
(43) Veröffentlichungstag der Anmeldung: 14.06.2017
(73) Patentinhaber: Fresenius Medical Care Deutschland GmbH, 61352 Bad Homburg (DE)
(72) Erfinder: GERLACH, Daniel, 60316 Frankfurt (DE); HÄCKER, Jürgen, 61267 Neu-Anspach (DE); NOACK, Joachim, 97616 Bad Neustadt (DE); SCHEUNERT, Peter, 61381 Friedrichsdorf (DE)
(74) Vertreter: Oppermann, Frank
(86) Internationale Anmeldenummer: PCT/EP2015/066687
(87) Internationale Veröffentlichungsnummer: WO 2016/020191

(56) Entgegenhaltungen:
- EP-A1- 0 319 279
- EP-A1- 2 221 075
- WO-A1-2010/066441
- WO-A2-2008/100989
- WO-A2-2010/102790
- WO-A2-2012/161744

## Beschreibung

Die Erfindung betrifft eine Blutbehandlungsvorrichtung mit einer Befestigungseinheit zur Befestigung einer zur einmaligen Verwendung bestimmten Funktionseinheit zur Durchführung der Blutbehandlung und eine Überwachungseinheit zur Überprüfung der Funktionsfähigkeit und/oder des Betriebszustandes der Funktionseinheit. Darüber hinaus betrifft die Erfindung ein Verfahren zum Überprüfen der Funktionsfähigkeit und/oder des Betriebszustandes einer Funktionseinheit, die zur Durchführung einer Blutbehandlung an einer Befestigungseinheit einer Blutbehandlungsvorrichtung befestigt wird.

Die bekannten Blutbehandlungsvorrichtungen verfügen über einen extrakorporalen Blutkreislauf und ein Dialysierflüssigkeitssystem. Es sind Blutbehandlungsvorrichtungen bekannt, bei denen Teile des extrakorporalen Blutkreislaufs und des Dialysierflüssigkeitssystems Bestandteil einer zur einmaligen Verwendung bestimmten Funktionseinheit (Disposable) sind. Diese Blutbehandlungsvorrichtungen weisen eine Befestigungseinheit auf, an der zur Durchführung der Blutbehandlung die Funktionseinheit befestigt wird.

Die DE 10 2009 018 664 A1 beschreibt eine externe Funktionseinheit für eine extrakorporale Blutbehandlungsvorrichtung. Die Funktionseinheit weist einen transparenten Gehäusekörper auf, in dem Kanäle und Kammern für Blut und Dialysierflüssigkeit ausgebildet sind. Die Zufuhr von Blut und Dialysierflüssigkeit erfolgt über Schlauchleitungen, die an Ein- und Auslässen des Gehäusekörpers angeschlossen sind. Die bekannten Funktionseinheiten werden auch als Kassetten bezeichnet.

Aus der DE 10 2009 012 633 A1 ist eine Befestigungseinheit zur Befestigung einer externen Funktionseinheit bekannt. Die Befestigungseinheit verfügt über einen feststehenden Befestigungsteil und einen schwenkbaren Befestigungsteil, wobei die Funktionseinheit zwischen dem feststehenden und schwenkbaren Befestigungsteil klemmend gehalten wird.

Die WO 2010/066441 A1 beschreibt eine Befestigungseinheit für eine Funktionseinheit einer extrakorporalen Blutbehandlungsvorrichtung, bei der die Funktionseinheit durch Erzeugung eines Unterdrucks an einer Anlagefläche der Befestigungseinheit gehalten wird.

Bei der Durchführung einer extrakorporalen Blutbehandlung besteht grundsätzlich das Risiko, dass Blut des Patienten aus dem extrakorporalen Blutkreislauf in das Dialysierflüssigkeitssystem gelangt. Dies ist bei einer Ruptur der semipermeablen Membran des Dialysators möglich. Daher wird bei den bekannten Blutbehandlungsvorrichtungen der Eintritt von Blut in das Flüssigkeitssystem überwacht. Die Erkennung von Blut (Hämoglobin) im Flüssigkeitssystem erfolgt nach dem Stand der Technik mittels eines fotometrischen Messverfahrens, bei dem die Abschwächung der Intensität einer Strahlung beim Durchgang durch die Dialysierflüssigkeit infolge des Eintritts von Blut erkannt wird. Ein spektroskopischer Blutleckdetektor ist beispielsweise aus der DE 10 2006 029 899 B4 bekannt.

Bei der Verwendung einer externen Funktionseinheit zur Durchführung einer Blutbehandlung besteht das Risiko des Eintritts von Blut in die Dialysierflüssigkeit oder das Substituat, wenn an Verbundstellen des Gehäusekörpers Undichtigkeiten auftreten. Beispielsweise können zwischen benachbarten Kammern und Kanälen des Gehäusekörpers Leckagen auftreten.

Aus der EP 0 319 279 A1 ist eine Behandlungsvorrichtung mit einer Befestigungseinheit zur Befestigung einer zur einmaligen Verwendung bestimmten Funktionseinheit und einer Überwachungseinheit zur Überprüfung der Funktionsfähigkeit oder des Betriebszustandes der Funktionseinheit bekannt. Die Behandlungsvorrichtung verfügt über ein optisches Sensormodul, das eine Ausnehmung aufweist, in die aber nur der rückwärtige Teil der Funktionseinheit eingesetzt ist. Am Boden des optischen Sensormoduls befinden sich ein Lichtsender und Lichtempfänger. Die Überwachungseinheit weist eine Auswerteeinheit auf, die derart konfiguriert ist, dass aus der Intensität des auf die Funktionseinheit fallenden Lichts und des reflektierten Lichts auf einen fehlerhaften Zustand oder einen bestimmten Betriebszustand der Funktionseinheit geschlossen werden kann.

Die WO 2010/100989 A2 beschreibt eine zur einmaligen Verwendung bestimmte Funktionseinheit zusammen mit einer Sensorik zur Überprüfung deren Funktionsfähigkeit, Die Sensorik umfasst eine Lichtquelle und eine Kamera, die auf beiden Seiten der Funktionseinheit angeordnet sind.

Aus der WO 2010/102790 A2 ist eine Aufnahmeeinheit für eine Funktionseinheit (Disposable-Kassette) bekannt, die einen ersten Kontaktabschnitt und einen zweiten Kontaktabschnitt für die Funktionseinheit umfasst. Der erste Kontaktabschnitt ist in einer rahmenförmigen Trägereinrichtung pendelnd oder drehbar gelagert, während der zweite Kontaktabschnitt an dem ersten Kontaktabschnitt in der Art einer Tür beweglich befestigt ist Der zweite Kontaktabschnitt dient der Abdeckung der Disposable-Kassette. Die WO 2010/102790 A2 schlägt vor, in dem ersten Kontaktabschnitt Aktoren oder Sensoren vorzusehen.

Der Erfindung liegt die Aufgabe zu Grunde, die Sicherheit der Blutbehandlung bei der Verwendung einer externen Funktionseinheit zur Durchführung der Blutbehandlung zu erhöhen. Insbesondere liegt der Erfindung die Aufgabe zu Grunde, eine Blutbehandlungsvorrichtung mit einer leicht zu handhabenden Befestigungseinheit zur Befestigung einer Funktionseinheit zur Durchführung der Blutbehandlung zu schaffen, die eine Überprüfung der Funktionsfähigkeit und/oder des Betriebszustandes der Funktionseinheit mit hoher Sicherheit erlaubt.

Die Lösung dieser Aufgaben erfolgt erfindungsgemäß mit den Merkmalen der unabhängigen Patentansprüche. Die Gegenstände der abhängigen Ansprüche betreffen vorteilhafte Ausführungsformen der Erfindung.

Die erfindungsgemäße Blutbehandlungsvorrichtung zeichnet sich durch eine Überwachungseinheit zur Überprüfung der Funktionsfähigkeit (Disposable) und/oder des Betriebszustandes der Funktionseinheit aus, die über mindestens einen Lichtsender und mindestens einen Lichtempfänger verfügt. Lichtsender und Lichtempfänger können Bestandteil einer Einrichtung zum Senden und Empfangen von Licht sein. Diese Einrichtung kann beispielsweise als LED-Array ausgebildet sein.

Lichtsender und Lichtempfänger sind bei der erfindungsgemäßen Überwachungseinheit auf einer Seite der Funktionseinheit angeordnet. Die Anordnung von Lichtsender und Lichtempfänger auf der gleichen Seite bietet den Vorteil, dass sich die Überwachungseinheit ohne größere bauliche Veränderungen in die Befestigungseinheit der Blutbehandlungsvorrichtung integrieren lässt.

Die Überwachung der Funktionseinheit beruht auf einem optischen Messverfahren, bei dem das an einem Teil der Funktionseinheit oder einem Teil der Befestigungseinheit reflektierte Licht erfasst wird. Die Rechen- und Auswerteinheit ist derart konfiguriert, dass das aus der Intensität des auf die Funktionseinheit fallenden Lichts und des an der Funktionseinheit oder Befestigungseinheit reflektierten Lichts auf einen fehlerhaften Zustand und/oder einen bestimmten Betriebszustand der Funktionseinheit geschlossen wird.

Wenn beispielsweise Blut von der Blutseite in die Dialysatseite aufgrund einer fehlerhaften Funktionseinheit eintreten sollte, kann der fehlerhafte Zustand mit der Funktionseinheit erkannt werden. Beispielsweise kann erkannt werden, ob Blut aus einer Blutkammer oder einem blutführenden Kanal in eine Dialysierflüssigkeitskammer oder einen Dialysierflüssigkeit oder Substituat führenden Kanal aufgrund einer Leckage in dem Gehäusekörper der Funktionseinheit eingetreten ist. Der Eintritt von Blut lässt sich nach den bekannten Verfahren zum Erkennen von Hämoglobin einfach nachweisen. Darüber hinaus kann erkannt werden, ob in einem Kanal oder einer Kammer der Funktionseinheit sich eine bestimmte Flüssigkeit, insbesondere Blut, Dialysierflüssigkeit oder Substituat befindet oder ob die Kammern oder Kanäle der Funktionseinheit überhaupt mit Flüssigkeit gefüllt sind. Die erfindungsgemäße Blutbehandlungsvorrichtung erlaubt somit nicht nur die Überprüfung der Funktionsfähigkeit, sondern auch des Betriebszustandes der Funktionseinheit.

Bei der erfindungsgemäßen Blutbehandlungsvorrichtung, die sich durch eine einfache Handhabbarkeit auszeichnet, verfügt die Befestigungseinheit über einen Befestigungsteil und einen Abdeckteil, die derart im Abstand zueinander angeordnet sind, dass die Funktionseinheit zwischen Befestigungsteil und Abdeckteil einsetzbar ist. Der Lichtsender und der Lichtempfänger sind an dem Abdeckteil vorgesehen.

Bei einer weiteren besonders bevorzugten Ausführungsform weist der Befestigungsteil der Befestigungseinheit eine das Licht des Lichtsenders reflektierende Anlagefläche für die Funktionseinheit auf. Das auf die Funktionseinheit fallende und durch die Funktionseinheit hindurchtretende Licht wird für die optische Reflektionsmessung an der Anlagefläche des Befestigungsteils reflektiert, so dass das Licht auf der gleichen Seite der Funktionseinheit ausgesandt und empfangen werden kann.

Der Abdeckteil der Befestigungseinheit ist zwischen einer geöffneten und einer geschlossenen Position beweglich an dem Befestigungsteil befestigt, so dass sich die Funktionseinheit einfach in die Befestigungseinheit einsetzen lässt. Vorzugsweise ist der Abdeckteil als eine Tür ausgebildet, die den Aufnahmeraum des Befestigungsteils zur Aufnahme der Funktionseinheit verschließt.

Der Abdeckteil der Befestigungseinheit kann als Klemmteil ausgebildet sein, so dass die Funktionseinheit zwischen Befestigungsteil und Abdeckteil klemmend befestigt werden kann.

Eine alternative Ausführungsform sieht vor, dass die Befestigungseinheit eine Saugeinheit zur Erzeugung eines Unterdrucks an dem Befestigungsteil aufweist, so dass die Funktionseinheit an dem Befestigungsteil durch eine Saugkraft befestigt werden kann. Bei dieser Ausführungsform weist die Anlagefläche des Befestigungsteils vorzugsweise ein flexibles Material auf, an dem die Funktionseinheit anliegt. Das flexible Material bildet vorzugsweise eine reflektierende Anlagefläche, an der das Licht reflektiert wird.

Die Vorteile der Anordnung von Lichtsender und -empfänger in bzw. an dem Abdeckteil liegen bei den bevorzugten Ausführungsformen insbesondere darin, dass sich das Befestigungsteil mit einer Anlagefläche ausbilden lässt, die nicht von Bauteilen des Lichtsenders und -empfängers durchbrochen ist. Insbesondere bei der Ausführungsform mit der ein flexibles Material aufweisenden Anlagefläche sind in dem flexiblen Material keine Öffnungen für den Durchtritt des Lichts erforderlich, die unter dem Gesichtspunkt einer einfachen Reinigung des flexiblen Materials nachteilig wären.

Darüber hinaus kommen die Vorteile der Erfindung insbesondere bei einer Funktionseinheit zum Tragen, bei der eine Kammer oder ein Kanal eine mit einer hydrophoben Membran verschlossene Öffnung aufweist, d.h. einer Funktionseinheit, in die ein oder mehrere Hydrophobfilter integriert sind. Bei einer derartigen Funktionseinheit erlaubt die Überwachungseinheit nicht nur die Überprüfung der Blut bzw. Dialysierflüssigkeit führenden Kanäle, sondern auch des Hydrophobfilters, durch den im Falle einer Ruptur Blut austreten kann. Zur Überwachung des Hydrophobfilters wird nicht das an der Befestigungseinheit reflektierte Licht, sondern das an einem Teil der Funktionseinheit reflektierte Licht, d.h. das an der hydrophoben Membran reflektierte Licht erfasst. Im Fehlerfall dringt Blut in die rückseitigen Fasern der Membran ein und färbt diese rot, so dass sich die Intensität des reflektierten Lichts ändert, was von der Überwachungseinheit erkannt wird.

Mit dem Lichtsender und Lichtempfänger der Überwachungseinheit kann auch erkannt werden, ob die Funktionseinheit an der Befestigungseinheit befestigt ist, da der Gehäusekörper der Funktionseinheit Licht absorbiert.

Für die unterschiedlichen Fälle können verschiedene Kriterien aufgestellt werden, so dass sich in Abhängigkeit von der Abschwächung des reflektierten Lichts auf die Funktionsfähigkeit und/oder den Betriebszustand der Funktionseinheit geschlossen werden kann.

Im Folgenden wird ein Ausführungsbeispiel der Erfindung unter Bezugnahme auf die Figuren im Einzelnen beschrieben.

Es zeigen:
- Fig. 1: eine Funktionseinheit zur Durchführung der Blutbehandlung für eine Blutbehandlungsvorrichtung in der Draufsicht und
- Fig. 2: die Befestigungseinheit zusammen mit der Funktionseinheit der Blutbehandlungsvorrichtung in stark vereinfachter schematischer Darstellung.

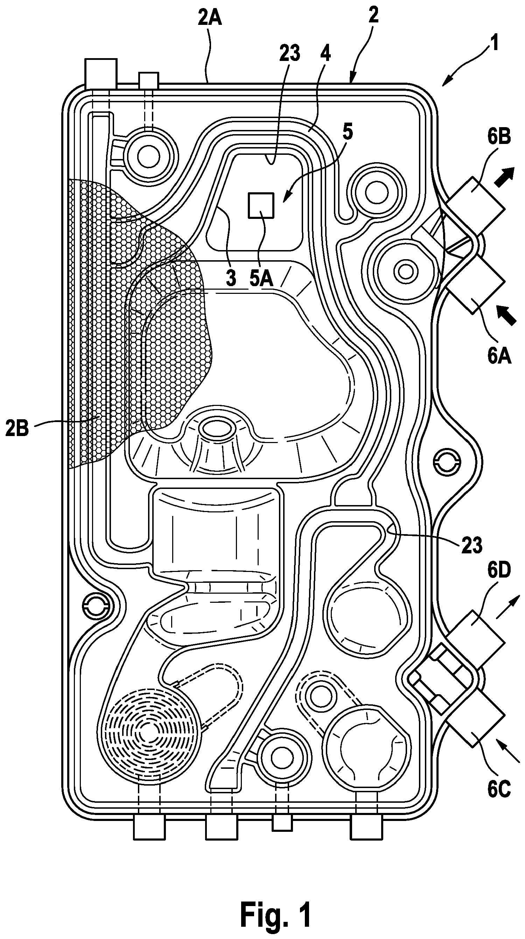

Fig. 1 zeigt ein Ausführungsbeispiel einer externen Funktionseinheit (Disposable) für eine extrakorporale Blutbehandlungsvorrichtung, insbesondere eine Hämodialysevorrichtung. Die Funktionseinheit 1 weist einen flachen Gehäusekörper 2 auf, der aus einem für Licht durchlässigen Material besteht. Der Gehäusekörper 2 kann ein transparenter Kunststoffkörper sein. Die Funktionseinheit ist im Einzelnen in der DE 10 2009 018 664 A1 beschrieben, auf die ausdrücklich Bezug genommen wird. Nachfolgend werden die für die Erfindung wesentlichen Komponenten der Funktionseinheit beschrieben.

Fig. 1 zeigt die Funktionseinheit (1) in der Unteransicht. Der Gehäusekörper 2 der Funktionseinheit 1 weist einen mit muldenförmigen Vertiefungen 23 geformten Gehäuseteil 2A auf, der an der Unterseite mit einer nur teilweise dargestellten Folie 2B unter Bildung einer Mehrzahl von Kammern und Kanälen dicht verschlossen ist. Während des Betriebs der Hämodialysevorrichtung befinden sich in den Kammern und Kanälen Blut oder eine medizinische Flüssigkeit, beispielsweise Dialysierflüssigkeit oder Substituat.

In Fig. 1 ist eine der Kammern mit dem Bezugszeichen 3 und einer der Kanäle mit dem Bezugszeichen 4 bezeichnet. In der Kammer 3 befindet sich während des Betriebs der Dialysevorrichtung Blut. Der Kanal 4 ist ein Kanal, in dem während des Betriebs der Dialysevorrichtung Substituat strömt. Darüber hinaus verfügt die Funktionseinheit 1 über einen Hydrophobfilter 5, der eine in Fig. 1 nur andeutungsweise dargestellte hydrophobe Membran 5A aufweist. Weiterhin verfügt die Funktionseinheit 1 noch über eine in Fig. 1 nicht näher bezeichnete Ventilanordnung, die eine Mehrzahl von Ventilen umfasst, mit denen die Flüssigkeitsverbindungen zwischen den einzelnen Kammern und Kanälen hergestellt werden können.

Das Blut und die medizinischen Flüssigkeiten werden über nicht dargestellte Schlauchleitungen zu- bzw. abgeführt, die an Anschlüssen 6A bis 6D des Gehäusekörpers 2 angeschlossen sind. Die nicht dargestellten Schlauchleitungen sind ggf. mit weiteren nicht dargestellten Komponenten Bestandteil der Funktionseinheit 1.

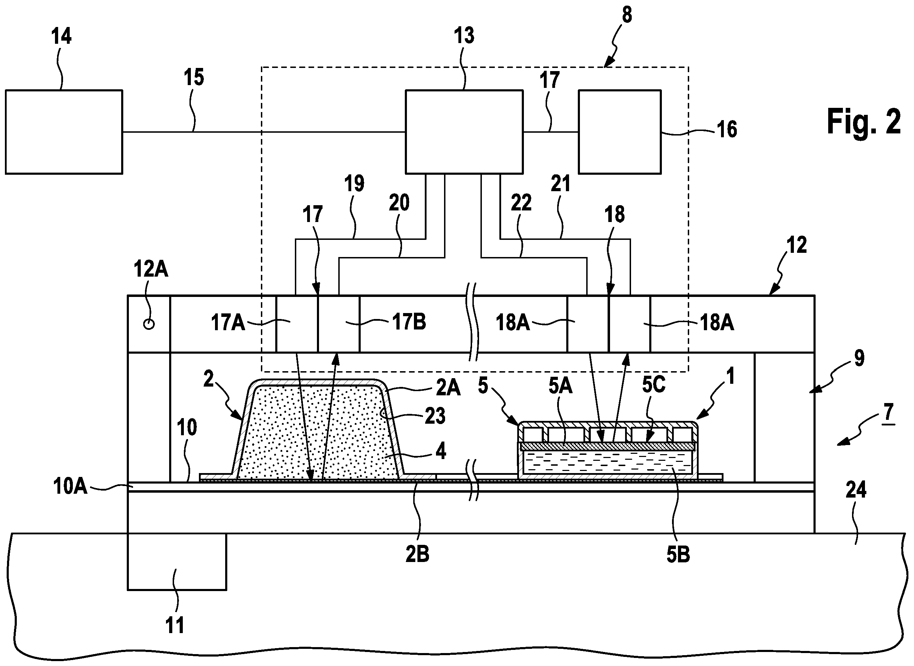

Fig. 2 zeigt in stark vereinfachter schematischer Darstellung den Teil des Gehäuses 24 der Blutbehandlungsvorrichtung, an dem sich eine Befestigungseinheit 7 zur Befestigung der Funktionseinheit 1 befindet. Fig. 2 zeigt nur die für die Erfindung wesentlichen Komponenten der Funktionseinheit 1 und der Befestigungseinheit 7. Darüber hinaus ist in Fig. 2 eine Überwachungseinheit 8 zum Überprüfen der Funktionsfähigkeit und/oder des Betriebszustandes der Funktionseinheit dargestellt.

Die Funktionseinheit 1 ist in die Befestigungseinheit 7 eingesetzt. Fig. 2 zeigt einen Schnitt der Funktionseinheit 1. In der Schnittdarstellung ist der Substituatkanal 4 und der Hydrophobfilter 5 mit der hydrophoben Membran 5A zu erkennen. Die übrigen Kammern und Kanäle sind der besseren Übersichtlichkeit halber nicht dargestellt.

Der Hydrophobfilter 5 umfasst eine Kammer 5B in dem Gehäusekörper 2, die an der Oberseite eine Öffnung 5C aufweist. Die Öffnung 5C der Kammer 5B ist mit der hydrophoben Membran 5A dicht verschlossen. Oberhalb der hydrophoben Membran 5C ist der Gehäusekörper 2 als Stützstruktur ausgebildet, so dass die hydrophobe Membran in dem Gehäusekörper fixiert ist. Während des Betriebs der Hämodialysevorrichtung, beispielsweise im Einnadel-Betrieb, ist die Kammer 5B des Hydrophobfilters 5 mit Blut befüllt.

Die Befestigungseinheit 7 der Blutbehandlungsvorrichtung weist einen maschinenseitigen Befestigungsteil 9 auf, der als ein an einer Seite offenes Gehäuse zur Aufnahme der Funktionseinheit 1 ausgebildet ist.

Bei der Durchführung der Blutbehandlung wird die Funktionseinheit 1 in den Befestigungsteil 9 eingelegt, wobei die Unterseite der Funktionseinheit 1 an einer Anlagefläche 10 des Befestigungsteils 9 anliegt. Die Befestigungseinheit 7 weist eine Saugeinheit 11 auf, die an der Anlagefläche 10 einen Unterdruck erzeugt, so dass die Funktionseinheit 1 an die Anlagefläche 10 angesaugt wird.

Darüber hinaus weist die Befestigungseinheit 7 einen Abdeckteil 12 auf, der um eine Achse 12A schwenkbar an dem Befestigungsteil 9 befestigt ist. Zum Einsetzen der Funktionseinheit wird der Abdeckteil 12 geöffnet und zum Betrieb der Blutbehandlungsvorrichtung geschlossen.

Nachfolgend wird die Überwachungseinheit 8 zur Überprüfung der Funktionsfähigkeit und/oder des Betriebszustandes der Funktionseinheit 1 beschrieben.

Die Überwachungseinheit 8 weist eine Rechen- und Auswerteinheit 13 auf, die auch Bestandteil der zentralen Steuereinheit 14 der Blutbehandlungsvorrichtung sein kann. Die Rechen- und Auswerteinheit 13 ist über eine Datenleitung 15 mit der zentralen Steuereinheit 14 verbunden, so dass die Rechen- und Auswerteinheit 13 Signale empfangen kann, die den Betriebszustand der Blutbehandlungsvorrichtung signalisieren. Mit der Rechen- und Auswerteinheit 13 ist über eine Datenleitung 17 eine Alarmeinheit 16 verbunden. Darüber hinaus weist die Überwachungseinheit 8 eine erste Sende- und Empfangseinheit 17 und eine zweite Sende- und Empfangseinheit 18 auf, die Licht aussenden bzw. empfangen.

Die erste und zweite Sende- und Empfangseinheit 17, 18 weisen jeweils einen Lichtsender 17A, 18A und einen Lichtempfänger 17B, 18B auf. Die Lichtsender und Lichtempfänger können Leuchtdioden sein (LED). Die Leuchtdioden der Lichtsender senden vorzugsweise Licht mit einer Wellenlänge aus, die dem Absorptionsmaximum von Hämoglobin entspricht. Dadurch ergibt sich eine maximale Empfindlichkeit der Überwachungseinheit 8 für die Erkennung von Blut.

Der Lichtsender und Lichtempfänger 17A, 17B der ersten Sende- und Empfangseinheit 17 ist an dem Abdeckteil 12 der Befestigungseinheit 7 oberhalb des Substituatkanals 4 angeordnet, während der Lichtsender und Lichtempfänger 18A, 18B der zweiten Sende- und Empfangseinheit 18 oberhalb der hydrophoben Membran 5A des Hydrophobfilters 5 in dem Abdeckteil 12 angeordnet ist.

Der Lichtsender 17A und der Lichtempfänger 17B der ersten Sende- und Empfangseinheit 17 sind derart angeordnet, dass das gesamte Licht des Lichtsenders 17A an der Anlagefläche 10 des Befestigungsteils 9 reflektiert wird und auf den Lichtempfänger 17B trifft. Bei einer bevorzugten Ausführungsform ist die Anlagefläche 10 des Befestigungsteils 9 mit einem flexiblen Material, beispielsweise einer Kunststoffmatte 10A belegt, an der die Funktionseinheit 1 anliegt. Die Kunststoffmatte 10A ist dabei derart beschaffen, dass die Lichtstrahlen reflektiert werden. Beispielsweise kann das flexible Material mit einer reflektierenden Beschichtung versehen sein.

Der Lichtsender 18A und der Lichtempfänger 18B der zweiten Sende- und Empfangseinheit 18 sind oberhalb des hydrophoben Filters 5 derart angeordnet, dass die Strahlen des Lichtsenders 18A von der hydrophoben Membran 5A reflektiert werden und auf den Lichtempfänger 18B treffen.

Die Rechen- und Auswerteinheit 13 empfängt die Signale der Lichtsender und -empfänger 17A, 17B; 18A, 18B über Datenleitungen 19, 20, 21, 22. Die Rechen- und Auswerteinheit 13 ist derart ausgebildet, dass auf der Grundlage einer Abschwächung der Intensität des reflektierten Lichts auf einen fehlerhaften und/oder einen bestimmten Betriebszustand der Funktionseinheit geschlossen werden kann, insbesondere auf eine Blutleckage geschlossen wird. Die Rechen- und Auswerteinheit 13 kann eine Blutleckage beispielsweise auf der Grundlage einer Messung erkennen, die auf dem Lambertschen Gesetz basiert. Derartige optische Messverfahren gehören zum Stand der Technik.

Wenn bei einer fehlerhaften Funktionseinheit 1 Blut in das Substituat gelangen sollte, wird das Licht des Lichtsenders 17A durch das Hämoglobin im Substituat absorbiert, so dass die Intensität des Lichts abgeschwächt wird. Wenn die Rechen- und Auswerteinheit 13 einen derartigen Störfall erkannt hat, erzeugt die Rechen- und Auswerteinheit 13 ein Alarmsignal, dass die Alarmeinheit 16 über die Datenleitung 17 empfängt. Darüber hinaus erzeugt die Rechen- und Auswerteinheit 13 ein Steuersignal, das die zentrale Steuereinheit 14 der Blutbehandlungsvorrichtung über die Datenleitung 15 empfängt, so dass die Steuereinheit 15 einen Eingriff in die Maschinensteuerung vornehmen kann.

Wenn die hydrophobe Membran 5A des Hydrophobfilters 5 defekt sein sollte, tritt Blut in die Membran ein, so dass sich die Membran rot färbt. Dadurch ändert sich die Intensität des reflektierten Lichts, das der Lichtempfänger 18B empfängt, so dass die Rechen- und Auswerteinheit 13 wieder ein Alarm- und Steuersignal für die Alarm- bzw. Steuereinheit 16, 14 erzeugt. Die Überwachungseinheit 8 erlaubt somit die Überwachung sämtlicher Bereiche der Funktionseinheit, an denen Leckagen auftreten können. Hierzu kann die Überwachungseinheit noch über weitere Sende- und Empfangseinheiten verfügen.

Neben der Funktionsfähigkeit überprüft die Überwachungseinheit 8 auch den Betriebszustand der Funktionseinheit. Wenn die Funktionseinheit 1 mit dem transparenten Gehäusekörper 2 in die Befestigungseinheit 7 eingelegt ist, ändert sich die Intensität des reflektierten Lichts. Die Rechen- und Auswerteinheit13 erkennt anhand des Signalpegels des Lichtempfängers 17B beispielsweise der ersten Sende- und Empfangseinheit 17, ob die Funktionseinheit eingelegt ist, indem die Rechen- und Auswerteinheit 13 den gemessenen Signalpegel mit einem vorgegebenen Referenzpegel vergleicht. Dieser Referenzpegel kann durch Kalibriermessungen festgelegt und in einem Speicher der Rechen- und Auswerteinheit 13 gespeichert werden.

Darüber hinaus kann die Rechen- und Auswerteinheit 13 erkennen, ob der Substituatkanal 4 mit Substituat gefüllt ist. Die Befüllung des Substituatkanals mit Substituat führt wieder zu einer Absorption des Lichts. Für die Messung kann ein weiterer Referenzpegel festgelegt werden, der in dem Speicher der Rechen- und Auswerteinheit 13 gespeichert wird. Es ist auch möglich, den Füllstand zu detektieren, wenn entsprechende Referenzpegel festgelegt werden.

Durch die Signalabfolge beim Einlegen, Ansaugen und Befüllen der Funktionseinheit 1 kann die Überwachungseinheit 8 getestet und/oder eingemessen werden. Hierzu kann die Rechen- und Auswerteinheit 13 die Steuersignale der zentralen Steuereinheit 14 empfangen, die den Betriebszustand der Blutbehandlungsvorrichtung signalisieren.

Bei einer besonders bevorzugten Ausführungsform wird die Sicherheit der Blutbehandlung dadurch erhöht, dass die einzelnen Betriebszustände der Blutbehandlungsvorrichtung in der Rechen- und Auswerteinheit 13 mit dem Betriebszustand der Funktionseinheit 1 verglichen werden. Dabei kann eine Plausibilitätsprüfung stattfinden. Beispielsweise kann mit der Rechen- uns Auswerteinheit 13 überprüft werden, ob die Steuereinheit 14 die Blutbehandlung in Gang setzen kann, was nur dann der Fall sein soll, wenn sich die Funktionseinheit 1 in der Befestigungseinheit 7 befindet. Die Steuereinheit 14 setzt die Blutbehandlungsvorrichtung nur dann in Gang, wenn sie von der Rechen- und Auswerteinheit 13 das entsprechende Steuersignal empfangen hat, das signalisiert, dass die Funktionseinheit ordnungsgemäß in die Befestigungseinheit eingesetzt ist.

## Patentansprüche

1. Blutbehandlungsvorrichtung mit einer Befestigungseinheit (7) zur Befestigung einer zur einmaligen Verwendung bestimmten Funktionseinheit (1) zur Durchführung der Blutbehandlung und einer Überwachungseinheit (8) zur Überprüfung der Funktionsfähigkeit und/oder des Betriebszustandes der Funktionseinheit, wobei die Überwachungseinheit (8) mindestens einen Lichtsender (17A, 18A) und mindestens einen Lichtempfänger (17B, 18B) aufweist, die derart angeordnet sind, dass das von dem Lichtsender (17A, 18A) ausgesandte Licht auf eine Seite der Funktionseinheit (1) fällt und das an einem Teil der Funktionseinheit oder einem Teil der Befestigungseinheit (7) reflektierte Licht auf den Lichtempfänger (17B, 18B) fällt, und die Überwachungseinheit (8) eine Rechen- und Auswerteinheit (13) aufweist, die derart konfiguriert ist, dass aus der Intensität des auf die Funktionseinheit (1) fallenden Lichts und des an der Funktionseinheit oder Befestigungseinheit (7) reflektierten Lichts auf einen fehlerhaften Zustand und/oder einen bestimmten Betriebszustand der Funktionseinheit (1) geschlossen wird,
**dadurch gekennzeichnet, dass**
die Befestigungseinheit (7) einen Befestigungsteil (9) und einen Abdeckteil (12) aufweist, wobei der Abdeckteil (12) zwischen einer geöffneten Position und einer geschlossenen Position beweglich an dem Befestigungsteil (9) befestigt ist und Befestigungsteil (9) und Abdeckteil (12) derart im Abstand zueinander angeordnet sind, dass die Funktionseinheit (1) zwischen Befestigungsteil (9) und Abdeckteil (12) einsetzbar ist, und
der Lichtsender (17A, 18A) und der Lichtempfänger (17B, 18B) in oder an dem beweglichen Abdeckteil (12) vorgesehen sind.

2. Blutbehandlungsvorrichtung nach Anspruch 1, **dadurch gekennzeichnet, dass** der Befestigungsteil (9) der Befestigungseinheit (7) eine das Licht des Lichtsenders (17A, 18A) reflektierende Anlagefläche (10) für die Funktionseinheit (1) aufweist.

3. Blutbehandlungsvorrichtung nach Anspruch 2, **dadurch gekennzeichnet, dass** die Anlagefläche (10) des Befestigungsteils (9) ein flexibles Material (10A) aufweist.

4. Blutbehandlungsvorrichtung nach einem der Ansprüche 1 bis 3, **dadurch gekennzeichnet, dass** der Abdeckteil (12) der Befestigungseinheit (7) als Klemmteil ausgebildet ist, so dass die Funktionseinheit (1) zwischen Befestigungsteil (9) und Abdeckteil (12) klemmend befestigbar ist.

5. Blutbehandlungsvorrichtung nach einem der Ansprüche 1 bis 3, **dadurch gekennzeichnet, dass** die Befestigungseinheit (7) eine Saugeinheit (11) zur Erzeugung eines Unterdrucks an dem Befestigungsteil (9) aufweist, so dass die Funktionseinheit (1) an dem Befestigungsteil (9) durch eine Saugkraft befestigbar ist.

6. Blutbehandlungsvorrichtung nach einem der Ansprüche 1 bis 5, **dadurch gekennzeichnet, dass** die Rechen- und Auswerteinheit (13) der Überwachungseinheit (8) derart konfiguriert ist, dass auf der Grundlage der Abschwächung der Intensität des Lichts darauf geschlossen wird, dass eine Funktionseinheit (1) an der Befestigungseinheit (7) befestigt ist.

7. Blutbehandlungsvorrichtung nach einem der Ansprüche 1 bis 6, **dadurch gekennzeichnet, dass** die Rechen- und Auswerteinheit (13) der Überwachungseinheit (8) derart konfiguriert ist, dass auf der Grundlage der Abschwächung der Intensität des Lichts darauf geschlossen wird, dass eine Kammer (3) oder ein Kanal (4) der Funktionseinheit (1) mit einer Flüssigkeit, insbesondere Dialysierflüssigkeit oder Substituat, befüllt ist.

8. Blutbehandlungsvorrichtung nach Anspruch 7, **dadurch gekennzeichnet, dass** die Rechen- und Auswerteinheit (13) der Überwachungseinheit (8) derart konfiguriert ist, dass ein Steuersignal erzeugt wird, wenn die Rechen- und Auswerteinheit (13) erkannt hat, dass die Kammer (3) und/oder der Kanal (4) der Funktionseinheit (1) mit einer Flüssigkeit, insbesondere Dialysierflüssigkeit oder Substituat, befüllt ist, wobei die Blutbehandlungsvorrichtung eine Steuereinheit (14) aufweist, die das Steuersignal der Rechen- und Auswerteinheit (13) empfängt.

9. Blutbehandlungsvorrichtung nach Anspruch 7 oder 8, **dadurch gekennzeichnet, dass** die Rechen- und Auswerteinheit (13) der Überwachungseinheit (8) derart konfiguriert ist, dass auf der Grundlage der Abschwächung der Intensität des Lichts darauf geschlossen wird, dass die Flüssigkeit, mit der die Kammer (3) oder der Kanal (4) befüllt ist, Hämoglobin enthält.

10. Blutbehandlungsvorrichtung nach Anspruch 9, **dadurch gekennzeichnet, dass** die Rechen- und Auswerteinheit (13) der Überwachungseinheit (8) derart konfiguriert ist, dass ein Alarmsignal erzeugt wird, wenn die Rechen- und Auswerteinheit (13) erkannt hat, dass die Flüssigkeit, mit der die Kammer oder der Kanal befüllt ist, Hämoglobin enthält, wobei die Blutbehandlungsvorrichtung eine Alarmeinheit (16) aufweist, die das Alarmsignal der Rechen- und Auswerteinheit (13) empfängt.

11. Anordnung mit einer Blutbehandlungsvorrichtung nach einem der Ansprüche 1 bis 10 und einer zur einmaligen Verwendung bestimmten Funktionseinheit (1) zur Durchführung der Blutbehandlung.

12. Anordnung nach Anspruch 11, **dadurch gekennzeichnet, dass** die Funktionseinheit (1) einen für Licht durchlässigen Gehäusekörper (2) aufweist, in dem mindestens eine Kammer (3; 5B) zur Aufnahme einer Flüssigkeit und/oder mindestens ein Kanal (4) zum Leiten einer Flüssigkeit ausgebildet sind, wobei der Lichtsender (17A, 18A) und der Lichtempfänger (17B, 18B) auf einer Seite des Gehäusekörpers (2) derart angeordnet sind, dass das von dem Lichtsender (17A, 18A) ausgesandte Licht auf eine Kammer (3; 5B) und/oder einen Kanal (4) der an der Befestigungseinheit (7) befestigten Funktionseinheit (1) fällt.

13. Anordnung nach Anspruch 12, **dadurch gekennzeichnet, dass** der Gehäusekörper (2) der Funktionseinheit (1) einen mit muldenförmigen Vertiefungen (23) geformten Gehäuseteil (2A) aufweist, der mit einem flachen Gehäuseteil (2B) flüssigkeitsdicht verschlossen ist.

14. Anordnung nach Anspruch 13, **dadurch gekennzeichnet, dass** der flache Gehäuseteil (2B) eine Folie ist, die mit dem mit muldenförmigen Vertiefungen (23) geformten Gehäuseteil (2A) verbunden ist.

15. Anordnung nach einem der Ansprüche 12 bis 14, **dadurch gekennzeichnet, dass** die Kammer (5B) oder der Kanal (4) eine mit einer hydrophoben Membran (5A) verschlossene Öffnung (5C) aufweist, wobei Lichtsender (17A, 18A) und Lichtempfänger (17B, 18B) derart angeordnet sind, dass das von dem Lichtsender (17A, 18A) ausgesandte Licht auf die hydrophobe Membran (5A) der Kammer (5B) fällt und das von der hydrophoben Membran (5A) reflektierte Licht auf den Lichtempfänger (17B, 18B) fällt.

16. Anordnung nach Anspruch 15, **dadurch gekennzeichnet, dass** die Rechen- und Auswerteinheit(13) der Überwachungseinheit (8) derart konfiguriert ist, dass auf der Grundlage der Abschwächung der Intensität des Lichts darauf geschlossen wird, dass die hydrophobe Membran (5A) defekt ist.

## Claims

1. Blood treatment device comprising an attachment unit (7) for attaching a functional unit (1) intended for single use for carrying out the blood treatment and a monitoring unit (8) for monitoring the operability and/or the operating state of the functional unit, the monitoring unit (8) comprising at last one light transmitter (17A, 18A) and at least one light receiver (17B, 18B), which are arranged such that the light emitted by the light transmitter (17A, 18A) falls on one side of the functional unit (1) and the light reflected on a part of the functional unit or a part of the attachment unit (7) falls on the light receiver (17B, 18B), and the monitoring unit (8) comprising a calculation- and evaluation unit 13 which is configured such that conclusions can be drawn as to a defective state and/or a certain operating state of the functional unit (1) based on the intensity of the light falling on the functional unit (1) and of the light reflected on the functional unit or the attachment unit (7).
**characterised in that**
the attachment unit (7) comprises an attachment component (9) and a cover component (12), the cover component (12) being attached to the attachment component (9) so as to be movable between an open position and a closed position, and attachment component (9) and cover component (12) are arranged at such a distance from one another that the functional unit (1) can be inserted between the attachment component (9) and the cover component (12), and
the light transmitter (17A, 18A) and the light receiver (17B, 18B) is provided in or on the cover component (12).

2. Blood treatment device according to claim 1, **characterised in that** the attachment component (9) of the attachment unit (7) comprises a contact surface (10) for the functional unit (1) that reflects the light of the light transmitter (17A, 18A).

3. Blood treatment device according to claim 2, **characterised in that** the contact surface (10) of the attachment component (9) comprises a flexible material (10A).

4. Blood treatment device according to any of claims 1 to 3, **characterised in that** the cover component (12) of the attachment unit (7) is configured as a clamp component, so that the functional unit (1) can be attached in a clamped manner between the attachment component (9) and the cover component (12).

5. Blood treatment device according to any of claims 1 to 3, **characterised in that** the attachment unit (7) comprises a suction unit (11) for creating a vacuum at the attachment component (9), so that the functional unit (1) can be attached to the attachment component (9) by way of suction power.

6. Blood treatment device according to any of claims 1 to 5, **characterised in that** the calculation- and evaluation unit (13) of the monitoring unit (8) is configured such that, based on the reduction of the intensity of the light, it can be concluded that a functional unit (1) is attached to the attachment unit (7).

7. Blood treatment device according to any of claims 1 to 6, **characterised in that** the calculation- and evaluation unit (13) of the monitoring unit (8) is configured such that, based on the reduction of the intensity of the light, it can be concluded that a chamber (3) or a channel (4) of the functional unit (1) is filled with a fluid, in particular dialysate or substitution fluid.

8. Blood treatment device according to claim 7, **characterised in that** the calculation- and evaluation unit (13) of the monitoring unit (8) is configured such that a control signal is generated when the calculation- and evaluation unit (13) has detected that the chamber (3) and/or the channel (4) of the functional unit (1) is filled with a fluid, in particular dialysate or substitution fluid, the blood treatment device comprising a control unit (14) which receives the control signal of the calculation- and evaluation unit (13).

9. Blood treatment device according to either claim 7 or claim 8, **characterised in that** the calculation- and evaluation unit (13) of the monitoring unit (8) is configured such that, based on the reduction of the intensity of the light, it can be concluded that the fluid with which the chamber (3) or the channel (4) is filled contains haemoglobin.

10. Blood treatment device according to claim 9, **characterised in that** the calculation- and evaluation unit (13) of the monitoring unit (8) is configured such that an alarm signal is generated when the calculation- and evaluation unit (13) has detected that the fluid with which the chamber or the channel is filled contains haemoglobin, the blood treatment device comprising an alarm unit (16) which receives the alarm signal of the calculation- and evaluation unit (13).

11. Arrangement comprising a blood treatment device according to any of claims 1 to 10 and a functional unit (1) intended for single use for carrying out the blood treatment.

12. Arrangement according to claim 11, **characterised in that** the functional unit (1) comprises a housing body (2) that is permeable to light, in which at least one chamber (3; 5B) for receiving a fluid and/or at least one channel (4) for carrying a fluid are formed, the light transmitter (17A, 18A) and the light receiver (17B, 18B) being arranged on one side of the housing body (2) such that the light emitted by the light transmitter (17A, 18A) falls on a chamber (3; 5B) and/or a channel (4) of the functional unit (1) that is attached to the attachment unit (7).

13. Arrangement according to claim 12, **characterised in that** the housing body (2) of the functional unit (1) comprises a housing part (2A) formed having trough-shaped recesses (23), which housing part is sealed by a flat housing part (2B) so as to be impermeable to fluids.

14. Arrangement according to claim 13, **characterised in that** the flat housing part (2B) is a film connected to the housing part (2A) formed having trough-shaped recesses (23).

15. Arrangement according to any of claims 12 to 14, **characterised in that** the chamber (5B) or the channel (4) comprises an opening (5C) sealed with a hydrophobic membrane (5A), the light transmitter (17A, 18A) and the light receiver (17B, 18B) being arranged such that the light emitted by the light transmitter (17A, 18A) falls on the hydrophobic membrane (5A) of the chamber (5B) and the light reflected by the hydrophobic membrane (5A) falls on the light receiver (17B, 18B).

16. Arrangement according to claim 15, **characterised in that** the calculation- and evaluation unit (13) of the monitoring unit (8) is configured such that, based on the reduction of the intensity of the light, it can be concluded that the hydrophobic membrane (5A) is defective.

## Revendications

1. Dispositif de traitement du sang avec une unité de fixation (7) servant à fixer une unité fonctionnelle (1) destinée à une utilisation unique servant à mettre en oeuvre le traitement du sang et une unité de surveillance (8) servant à vérifier le bon fonctionnement et/ou l'état de fonctionnement de l'unité fonctionnelle, dans lequel l'unité de surveillance (8) présente au moins un émetteur de lumière (17A, 18A) et au moins un récepteur de lumière (17B, 18B), qui sont disposés de telle manière que la lumière envoyée par l'émetteur de lumière (17A, 18B) est incidente sur un côté de l'unité fonctionnelle (1) et la lumière réfléchie au niveau d'une partie de l'unité fonctionnelle ou d'une partie de l'unité de fixation (7) est incidente sur le récepteur de lumière (17B, 18B), et l'unité de surveillance (8) présente une unité de calcul et d'évaluation (13), qui est configurée de telle manière qu'il est conclu à partir de l'intensité de la lumière incidente sur l'unité fonctionnelle (1) et de la lumière réfléchie au niveau de l'unité fonctionnelle ou de l'unité de fixation (7), à un état défectueux et/ou à un état de fonctionnement donné de l'unité fonctionnelle (1),
**caractérisé en ce que**
l'unité de fixation (7) présente une partie de fixation (9) et une partie de recouvrement (12), dans lequel la partie de recouvrement (12) est fixée entre une position ouverte et une position fermée de manière mobile au niveau de la partie de fixation (9) et la partie de fixation (9) et la partie de recouvrement (12) sont disposées de telle manière à distance l'une par rapport à l'autre que l'unité fonctionnelle (1) peut être insérée entre la partie de fixation (9) et la partie de recouvrement (12), et
l'émetteur de lumière (17A, 18A) et le récepteur de lumière (17B, 18B) sont prévus dans ou au niveau de la partie de recouvrement (12) mobile.

2. Dispositif de traitement du sang selon la revendication 1, **caractérisé en ce que** la partie de fixation (9) de l'unité de fixation (7) présente une surface d'appui (10), réfléchissant la lumière de l'émetteur de lumière (17A, 18A), pour l'unité fonctionnelle (1).

3. Dispositif de traitement du sang selon la revendication 2, **caractérisé en ce que** la surface d'appui (10) de la partie de fixation (9) présente un matériau (10A) flexible.

4. Dispositif de traitement du sang selon l'une quelconque des revendications 1 à 3, **caractérisé en ce que** la partie de recouvrement (12) de l'unité de fixation (7) est réalisée sous la forme d'une partie de serrage de sorte que l'unité fonctionnelle (1) peut être fixée par serrage entre la partie de fixation (9) et la partie de recouvrement (12).

5. Dispositif de traitement du sang selon l'une quelconque des revendications 1 à 3, **caractérisé en ce que** l'unité de fixation (7) présente une unité d'aspiration (11) servant à générer une dépression au niveau de la partie de fixation (9) de sorte que l'unité fonctionnelle (1) peut être fixée au niveau de la partie de fixation (9) par une force d'aspiration.

6. Dispositif de traitement du sang selon l'une quelconque des revendications 1 à 5, **caractérisé en ce que** l'unité de calcul et d'évaluation (13) de l'unité de surveillance (8) est configurée de telle manière qu'il est conclu sur la base de l'affaiblissement de l'intensité de la lumière qu'une unité fonctionnelle (1) est fixée au niveau de l'unité de fixation (7).

7. Dispositif de traitement du sang selon l'une quelconque des revendications 1 à 6, **caractérisé en ce que** l'unité de calcul et d'évaluation (13) de l'unité de surveillance (8) est configurée de telle manière qu'il est conclu sur la base de l'affaiblissement de l'intensité de la lumière qu'une chambre (3) ou un canal (4) de l'unité fonctionnelle (1) est rempli(e) d'un liquide, en particulier d'un liquide de dialyse ou d'une solution de substitution.

8. Dispositif de traitement du sang selon la revendication 7, **caractérisé en ce que** l'unité de calcul et d'évaluation (13) de l'unité de surveillance (8) est configurée de telle manière qu'un signal de commande est généré quand l'unité de calcul et d'évaluation (13) a identifié que la chambre (3) et/ou le canal (4) de l'unité fonctionnelle (1) est rempli(e) d'un liquide, en particulier d'un liquide de dialyse ou d'une solution de substitution, dans lequel le dispositif de traitement du sang présente une unité de commande (14), qui reçoit le signal de commande de l'unité de calcul et d'évaluation (13).

9. Dispositif de traitement du sang selon la revendication 7 ou 8, **caractérisé en ce que** l'unité de calcul et d'évaluation (13) de l'unité de surveillance (8) est configurée de telle manière qu'il est conclu sur la base de l'affaiblissement de l'intensité de la lumière que le liquide, avec lequel la chambre (3) ou le canal (4) est rempli(e), contient de l'hémoglobine.

10. Dispositif de traitement du sang selon la revendication 9, **caractérisé en ce que** l'unité de calcul et d'évaluation (13) de l'unité de surveillance (8) est configurée de telle manière qu'un signal d'alarme est généré quand l'unité de calcul et d'évaluation (13) a identifié que le liquide, avec lequel la chambre ou le canal est rempli(e), contient de l'hémoglobine, dans lequel le dispositif de traitement du sang présente une unité d'alarme (16), qui reçoit le signal d'alarme de l'unité de calcul et d'évaluation (13).

11. Ensemble avec un dispositif de traitement du sang selon l'une quelconque des revendications 1 à 10 et une unité fonctionnelle (1) destinée à une utilisation unique servant à mettre en oeuvre le traitement du sang.

12. Ensemble selon la revendication 11, **caractérisé en ce que** l'unité fonctionnelle (1) présente un corps de boîtier (2) laissant passer la lumière, dans lequel au moins une chambre (3 ; 5B) servant à recevoir un liquide et/ou au moins un canal (4) servant à acheminer un liquide sont réalisés, dans lequel l'émetteur de lumière (17A, 18A) et le récepteur de lumière (17B, 18B) sont disposés sur un côté du corps de boîtier (2) de telle manière que la lumière envoyée par l'émetteur de lumière (17A, 18A) est incidente sur une chambre (3 ; 5B) et/ou un canal (4) de l'unité fonctionnelle (1) fixée au niveau de l'unité de fixation (7).

13. Ensemble selon la revendication 12, **caractérisé en ce que** le corps de boîtier (2) de l'unité fonctionnelle (1) présente une partie de boîtier (2A) façonnée avec des renfoncements (23) en forme de cavité, qui est fermée de manière étanche aux liquides avec une partie de boîtier plate (2B).

14. Ensemble selon la revendication 13, **caractérisé en ce que** la partie de boîtier plate (2B) est un film, qui est relié à la partie de boîtier (2A) façonnée avec des renfoncements (23) en forme de cavité.

15. Ensemble selon l'une quelconque des revendications 12 à 14, **caractérisé en ce que** la chambre (5B) ou le canal (4) présente une ouverture (5C) fermée avec une membrane hydrophobe (5A), dans lequel l'émetteur de lumière (17A, 18A) et le récepteur de lumière (17A, 18B) sont disposés de telle manière que la lumière envoyée par l'émetteur de lumière (17A, 18A) est incidente sur la membrane hydrophobe (5A) de la chambre (5B) et la lumière réfléchie par la membrane hydrophobe (5A) est incidente sur le récepteur de lumière (17B, 18B).

16. Ensemble selon la revendication 15, **caractérisé en ce que** l'unité de calcul et d'analyse (13) de l'unité de surveillance (8) est configurée de telle manière qu'il est conclu sur la base de l'affaiblissement de l'intensité de la lumière que la membrane hydrophobe (5A) est défectueuse.
